(19) Europäisches Patentamt
European Patent Office
Office européen des brevets

(11) **EP 3 025 700 A1**

(12) **EUROPÄISCHE PATENTANMELDUNG**

(43) Veröffentlichungstag:
01.06.2016  Patentblatt 2016/22

(21) Anmeldenummer: 14195302.6

(22) Anmeldetag: **28.11.2014**

(51) Int Cl.:
*A61K 8/25* (2006.01)   *A61Q 17/00* (2006.01)
*A61Q 17/04* (2006.01)   *C01B 33/027* (2006.01)
*C01B 33/029* (2006.01)   *C01B 33/03* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Evonik Degussa GmbH**
**45128 Essen (DE)**

(72) Erfinder:
• **Lang,Dr. Jürgen Erwin**
  **76229 Karlsruhe (DE)**
• **Kröll, Dr. Michael**
  **63589 Linsengericht (DE)**
• **Uehlenbruck, Goswin**
  **61440 Oberursel (DE)**

(54) **Verwendung von Silicium enthaltenden Partikeln zum Schutz vor UV-Strahlung, Verfahren zu deren Herstellung sowie Formulierungen enthaltend diese**

(57)    Die Erfindung betrifft die Verwendung von Silicium enthaltenden Partikeln zum Schutz von humanen Zellen vor elektromagnetischer Strahlung im UV-Bereich und optional im Vis- bis in den IR-Bereich, wobei die Partikel vorzugsweise als Cluster von Primärpartikeln mit einer Partikelgröße im Bereich von 5 bis 100 nm vorliegen. Ein besondere Vorteil der erfindungsgemäßen Verwendung besteht in der Möglichkeit die Absorption der elektromagnetischen Strahlung über die Partikelgröße definiert an die zu absorbierenden Wellenlängenbereich anzupassen. Die Silicium enthaltenden Partikel können als bioverträgliche und biologisch abbaubarer UV-Schutz in kosmetischen oder medizinischen Formulierungen, wie vorzugsweise einer Sonnencreme oder auch in einer kosmetischen Formulierung für den UV-Schutz von Haaren, verwendet werden.

EP 3 025 700 A1

**Beschreibung**

Die Erfindung betrifft die Verwendung von Silicium enthaltenden Partikeln zum Schutz von humanen Zellen vor elektromagnetischer Strahlung im UV-Bereich und optional im Vis- bis in den IR-Bereich, wobei die Partikel vorzugsweise als Cluster von Primärpartikeln mit einer Partikelgröße im Bereich von 5 bis 100 nm vorliegen. Ein besonderer Vorteil der erfindungsgemäßen Verwendung besteht in der Möglichkeit die Absorption der elektromagnetischen Strahlung über die Partikelgröße definiert an die zu absorbierenden Wellenlängenbereich anzupassen. Die Silicium enthaltenden Partikel, insbesondere aus Silicium bestehenden Partikeln, können als bioverträgliche und biologisch abbaubarer UV-Schutz in kosmetischen oder medizinischen Formulierungen, wie vorzugsweise einer Sonnencreme oder auch in einer kosmetischen Formulierung für den UV-Schutz von Haaren, verwendet werden.

Bekannte UV-Schutzformulierungen basieren auf UV-Filtern unterschiedlicher Konzentration. Die verwendeten UV-Filter können dabei nach unterschiedlichen Mechanismen wirken. Die bekannten UV-Filter weisen dabei ein typische Absorptions-spektrum auf und müssen, da sie meist nicht über das gesamte Spektrum wirken mit anderen UV-Filtern kombiniert werden. Somit basieren die Sonnencremes häufig auf Mischungen verschiedener UV-Filter. In den Sonnencremes wirkten die UV-Filter zur Beschichtung der Haut und ziehen teilweise in die Hornschicht der Haut ein.

Übliche UV-Filter, wie organische UV-Filter, wirken durch eine Stokes-Verschiebung des absorbierten Lichts in den längerwelligen Wellenlängenbereich. Die UV-Filter werden nach ihrem Absorptionsspektrum als UVA-, UVB- und Breitbandfilter (UVA-/ UVB-Filter) klassifiziert. Organische UV-Filter sind oftmals Derivate von beispielsweise Campher oder Salicylsäure. Des Weiteren sind anorganische UV-Filter bekannt, wie feinteilige Titandioxid- und Zinkoxid-Partikel. Ein Vorteil und zugleich ein Nachteil dieser Partikel ist ihre Sichtbarkeit. Einerseits kann über die Sichtbarkeit eine Auftragskontrolle erfolgen, so dass diese UV-Filter in der Regel bei Sonnenschutzprodukten für Kinder Anwendung finden. Andererseits ist die Akzeptanz von sichtbaren UV-Filtern bei Produkten für Erwachsene sehr umstritten.

Ein weiterer Nachteil anorganischer UV-Filter wie Zinkoxid ist häufig die geringe Partikelgröße, die ein Eindringen in die Haut ermöglichen kann. Geringe Mengen der Zinkpartikel wurden im Blut und im Urin nachgewiesen. So sind in der Schweiz keine nanopartikulären Zinkoxid basierte Sonnenschutzprodukte zugelassen und auch in Deutschland darf die Konzentration einen festgelegten Wert an nanopartikulären Zinkoxid nicht überschreiten.

Es besteht daher ein Bedarf an neuen UV-Filtern, die die vorgenannten Nachteile nicht aufweisen. Vorzugsweise sollen die UV-Filter auf einem anorganischen Material basieren. Weiter ist es bevorzugt, wenn die UV-Filter sich durch die Einwirkung von UV-Strahlung nicht zersetzen und keine biounverträglichen Zersetzungs- oder Abbauprodukte bilden. Des Weitern sollen die UV-Filter nicht resorbiert werden oder bei einer Resorption im Organismus abbaubar sein.

Des Weitern bilden Silane, insbesondere hochreine Silane, eine wichtige Produktklasse in vielen Anwendungsgebieten, wie der Halbleiterindustrie oder Lichtwellenleiter-industrie. In der Kosmetikindustrie sind weitgehend nur Siliconöle oder Siloxane als Additive bekannt. Diese Anwendungsbereiche stellen hohe Anforderungen an die Reinheit dieser Substanzen, insbesondere in Bezug auf Spurenkontaminationen sowie Verträglichkeit auf der Haut.

Als besonders negativ erweisen sich im Bereich der Kosmetik, und hier insbesondere im Bereich des Sonnenschutzes Kontaminationen mit allergenen Verbindungen, z. B. Nickel bzw. andere herstellungsbedingten Katalysatoren oder mit $TiO_2$ das photokatalytisch wirken kann. Solche Kontaminationen können selbst im unteren %-Bereich und sogar bis ppm (Gew.) Bereich äußerst störend in den Kosmetik-Anwendungen sein.

Es bestand daher die Aufgabe, einen neuen UV-Filter zu entwickeln. Der neue UV-Filter soll sich nicht durch UV-Strahlung zersetzten und soll vorzugsweise biologisch abbaubar oder im Organismus abbaubar sein. Ferner soll der neue UV-Filter durch ein umweltfreundliches und energetisch verbessertes Verfahren herstellbar sein. Vorteilhaft soll auf die Verwendung von Katalysatoren verzichtet werden. Ferner soll die Anzahl an Aufbereitungsschritten vermindert werden. Des Weiteren bestand die Aufgabe einen Prozess zu finden, der kontinuierlich oder satzweise hohe Durchsätze der herzustellenden Verbindungen erlaubt.

Völlig überraschend und unerwartet konnten die Aufgaben gelöst werden, indem ein Silan-Massenstrom plasma-thermisch behandelt wird. Der Silan-Massenstrom, vorzugsweise enthaltend Monosilan $SiH_4$, wird zusammen mit einer Kontamination, bei der es sich erfindungsgemäß um Silizium-Halogenverbindungen, z. B. Chlor, Brom enthaltenden Verbindungen handelt, einer Plasmaentladungsanordnung zugeführt und dort beispielhaft in einer Hochspannungs-Pulsentladung zur Reaktion gebracht.

Der neue Verfahrensansatz besteht darin, den Massestrom dahingehend zu behandeln, dass er durch eine Partikelgrößenverteilung charakterisiert werden kann, über die die spektralen Absorptionseigenschaften im UV-Bereich und im Vis-Bereich sowie optional im IR-Bereich eingestellt werden können. Hinsichtlich der Wirkung der Gasentladungsbehandlung wird angenommen, dass eine Begünstigung der Kinetik erfolgt, wenn plasmachemisch Silizium-H oder Si-Cl-Radikale angeregt werden und diese selektiv und bevorzugt Siliziumpartikel kleiner 100 nm bilden, wodurch erfindungsgemäß eine Absorptionsverschiebung aus dem UV-C (100-280 nm), über UV-B (280 bis 315 nm) bis zum UV-A (315 bis 380 nm) resultiert. In Abhängigkeit von der mittleren Primärpartikelgröße kann die Absorption im UV-C- und UV-B-Bereich gegenüber der Absorption im UV-A-Bereich gesteigert werden.

Der Massestrom ist im erfindungsgemäßen Verfahren einer einfachen Aufarbeitung, wie Filtration und/oder Wäsche, zugänglich, wodurch das Reinprodukt bevorzugt abgezogen wird. Anfallendes Restgas, das neben $H_2$ auch Spuren von HCl enthält, wird zurückgeführt. Für die bekannten Prinzipien der Gasentladung und der Plasma-Chemie wird auf die einschlägige Fachliteratur verwiesen: beispielsweise von A.T. Bell "Fundamentals of Plasma Chemistry" ed. J.R. Hollahan und A.T. Bell, Wiley, New York (1974).

Die Aufgaben konnten ebenfalls in überraschender Weise gelöst werden, indem Silicium enthaltende Partikel durch Umsetzung von Monosilan oder höheren H-Silanen, wie der Formel $MeH_n$ oder $Me_2H_{2n-2}$, wobei Me für Silicium und n für eine ganze Zahl steht, Halogensilanen, höheren Halogensilanen oder Alkoxysilanen optional mit C oder N oder Ge enthaltenden Spezies in einem thermischen Prozess, ggf. kombiniert mit einem Plasmaprozess oder in einem reinen Plasmaprozess umgesetzt werden. Höhere H-Silane oder Chlorsilane werden auch als Polysilane oder Polychlorsilane bezeichnet. Die höheren H-Silane oder höheren Chlorsilane werden vor Ihrer Umsetzung in die Gasphase überführt. Das Halogen kann ausgewählt sein aus Fluor, Chlor, Brom oder Iod, bevorzugt ist Chlor.

Erfindungsgemäß werden die Aufgaben ebenso gelöst durch die erfindungsgemäße Verwendung von Silicium enthaltenden Partikeln, insbesondere von amorphen, reinen Silicium enthaltenen Partikeln, indem die Partikel zum Schutz von Zellen vor elektromagnetischer Strahlung im Wellenlängenbereich von 10 bis 2500 nm, insbesondere bis 1500 nm verwendet werden. Insbesondere werden die Partikel zum Schutz von Zellen vor einer Schädigung durch UV-Strahlung verwendet. Die geschützten Zellen umfassen humane, tierische und oder auch pflanzliche Zellen, wobei Zelle der Epidermis, wie Hautzellen, Hornhautzellen oder auch Zellen der Haare bevorzugt vor einer Beeinflussung durch elektromagnetische Strahlung geschützt werden. Die Zellen müssen dabei nicht unmittelbar mit den Partikel in Kontakt kommen, sondern könne auch mittelbar geschützt werden, indem die Partikel beispielsweise auf Gläsern von Sonnenbrillen oder zur Ausrüstung von textilen Materialien verwendet werden oder im Zusammenhang mit wissenschaftlichen oder industriellen Anwendungen, die dem Schutz von Zellen, beispielsweise von Bakterienkulturen dienen.

Bevorzugte reine Silicium enthaltende Partikel, insbesondere Primärpartikel, die vorzugsweise in Clustern vorliegen, weisen einen Gehalt von Silicium von größer gleich 50 Gew.-% bis 100 Gew.-% in Bezug auf die Gesamtzusammensetzung an Silicium enthaltenden Partikeln auf. Insbesondere beträgt der Gehalt an Silicium größer gleich 55 Gew.-%, bevorzugt beträgt der Siliciumanteil größer 80 Gew.-% , 90 Gew.-%, 95 Gew.-%, 98 Gew.-%, 99 Gew.-%, 99,5 Gew.-%, 99,99 Gew.-%, 99,999 Gew.-% bis 100,0 Gew.-%, und optional weisen die Partikel auf den Siliziumanteil in Gew.-% einen Gehalt an Kohlenstoff und/oder Sauerstoff auf, wobei vorzugsweise die Partikel im Wesentlichen amorph sind. Der Gehalt an Sauerstoff liegt kleiner 50 Gew.-% bevorzugt kleiner 10 Gew.-%, besonders bevorzugt kleiner 1 Gew.-%. Besonders bevorzugt sind reine Silicium enthaltende Partikel, insbesondere Primärpartikel, die vorzugsweise in Clustern vorliegen, mit einem Gehalt an Silicium von größer gleich 90 Gew.-% bis 100 Gew.-% in Bezug auf die Gesamtzusammensetzung an Silicium enthaltenden Partikeln. Ferner können die Silicium enthaltenden Partikel von 0 bis 10 Gew.-% Selen als Radikalfänger umfassen. Bevorzugt können die Partikel 0,0001 bis 5 Gew.-% Selen umfassen.

Ebenso Gegenstand der Erfindung ist die Verwendung der Silicium enthaltenden Partikel a) zur Absorption von elektronmagnetischer Strahlung im Wellenlängenbereich von größer gleich 10 nm bis 1100 nm, insbesondere im Wellenlängenbereich von 10 nm bis 450 nm. Bevorzugt werden die Silicium enthaltenden Partikel als UV-Schutz vor elektromagnetischer Strahlung verwendet, vorzugsweise als UV-Schutz von 180 bis 400 nm, besonders bevorzugt von 200 bis 380 oder bis 400 nm. Ebenso bevorzugt ist, dass die Silicium enthaltenden Partikel auch vor elektromagnetischer Strahlung im Wellenlängenbereich von des extremen UV wie 10 bis 100 nm bzw. bis 120 nm, im fernen UV, von 200 bis 280 nm, im mittleren UV von 280 bis 315 nm, und/oder im nahen UV von 315 bis 380 sowie je nach Partikelgrößer der Primärpartikel von 400 bis 750 nm im Vis-Bereich sowie optional im IR-Bereich oberhalb von 750 nm bis ca. 1500 nm von Zellen vor elektromagnetischer Strahlung schützen können. Die definierte Absorption in den vorgenannten Bereichen kann spezifisch über den Gehalt der jeweiligen mittleren Primärpartikelgrößen gezielt eingestellt werden.

Es hat sich gezeigt, dass sich die spektroskopischen Eigenschaften der amorphen Partikel auch direkt über das Herstellverfahren hinsichtlich der Größenverteilung der Primärpartikel und/oder Clusterbildung, sowie optional über einen Zusatz von Kohlenstoff, Stickstoff, Germanium oder weiteren Silanen einstellen lassen.

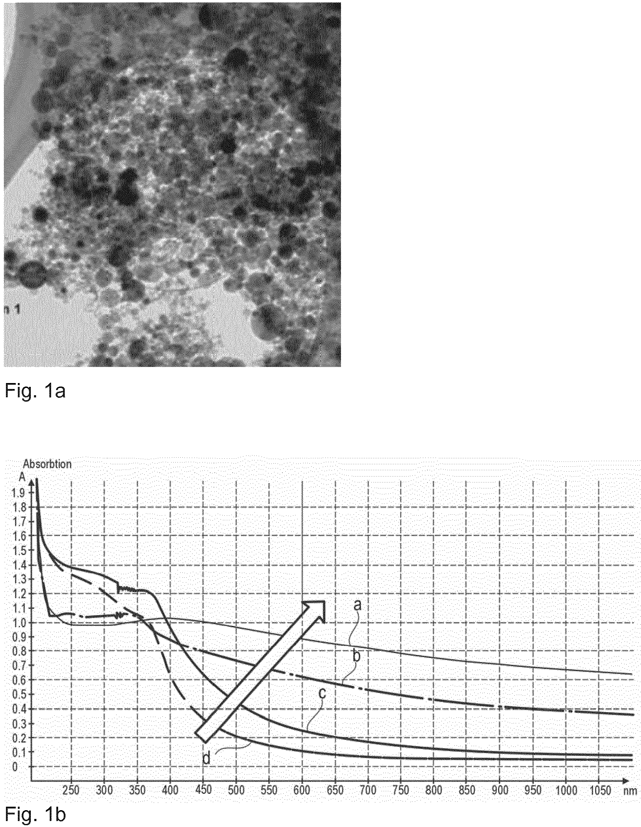

So konnte die Absorption über die Partikelgröße der Silicium enthaltenden Partikel, die Primärpartikel und Cluster der Primärpartikel umfassen, unmittelbar eingestellt werden. Überraschend absorbieren Partikel mit einer Primärpartikelgröße von 5 bis 80 nm und unterschiedlichen mittleren Partikelgrößen von $d_{50}$ um 10 nm, 15, 20, 25, 30 35 und 40 nm im UV-und im Vis-Bereiche deutlich unterschiedlich. Siliciumpartikel mit einer Partikelgröße der Primärpartikel von im Mittel $d_{50}$ 15 bis 30 nm, vorzugsweise 25 nm bis 30 nm, insbesondere mit $d_{90}$ 3 bis 50 nm, absorbieren deutlich stärker im UV-Bereich als im Vis-Bereich, während Partikel mit einer Primärpartikelgröße von im Mittel von 35 bis 40 nm, insbesondere mit $d_{90}$ 10 bis 50 nm, im UV/Vis-Bereich vergleichbar stark absorbieren (Figur 1 b).

Besonders bevorzugt ist die Verwendung von Silicium enthaltenden Partikeln, die im Wesentlichen amorphe Partikel sind. Als amorph gelten Partikel, die eine Kristallinität von kleiner gleich 2 % aufweisen. Vorzugsweise weisen die amorphen Silicium enthaltenden Partikel einen Primärpartikelgröße von im Mittel $d_{50}$ 20 nm, 25 m, 30 nm oder 40 nm, vorzugsweise jeweils unabhängig mit einer geringen Streuung von +/- 10 nm, insbesondere +/- 5 nm für $d_{90}$ auf.

Die Cluster der Primärpartikel können 30 bis 400 nm groß sein, wobei die Cluster übliche Größen von im Mittel um 150 nm mit plus/minus 50 nm oder alternativ von 50 bis 100 nm aufweisen.

Erfindungsgemäße Silicium enthaltende Partikel mit Primärpartikeln mit einer mittleren Partikelgröße der Primärpartikel von 10 bis 30 nm, wie vorzugsweise um $d_{50} = 13,2$ nm und vorzugszugsweise $d_{20} = 7,2$ nm und $d_{90} = 23,5$ nm, weisen im Wellenlängenbereich von 180 bis 1000 nm eine Transparenz von kleiner 40 % (Absorption größer gleich 0,6), insbesondere bei 180 bis 700 nm eine Transparenz von kleiner gleich 20 % (Absorption größer gleich 0,8), vorzugsweise zudem von 180 bis 475 nm eine Transparenz kleiner gleich 5 % (Absorption größer 0,95) auf, wobei vorzugsweise die Primärpartikel Cluster mit größer gleich 50 nm bilden und amorph sind.

Alternative erfindungsgemäße Silicium enthaltende Partikel mit Primärpartikeln mit einer mittleren Partikelgröße der Primärpartikel von 35 bis 40 nm, weisen im Wellenlängenbereich von 180 bis 400 nm eine Transparenz von kleiner 40 %, gleichbedeutend mit einer Absorption von größer oder gleich 0,6, insbesondere bei 180 bis 350 nm eine Transparenz von kleiner gleich 0 % (Absorption größer gleich 1,0), vorzugsweise zudem von 180 bis 300 nm eine Transparenz kleiner gleich 0 % (Absorption größer 1,2) auf, wobei vorzugsweise die Primärpartikel Cluster mit im Mittel 30 bis 400 nm, insbesondere um 150 nm mit plus/minus 50 nm bilden und amorph sind.

Ferner ist Gegenstand der Erfindung die Verwendung von Silicium enthaltenden Partikel mit einem Gehalt von größer gleich 40 Gew.-%, insbesondere größer gleich 50 bis 100 Gew.-% Silicium und einer Partikelgröße, insbesondere Primärpartikelgröße, von 1 bis 500 nm. Vorzugsweise beträgt der Gehalt an Silicium in der Gesamtzusammensetzung der Silicium enthaltenden Partikeln oder in den jeweiligen einzelnen Partikeln, 70 Gew.-%, wie SiC, bis 100 Gew.-%, insbesondere 80 bis 100 Gew.-%, vorzugsweise 90 bis 100 Gew.-%, besonders bevorzugt 99,5 bis 100 Gew.-% an Silicium sowie optional zusätzlich mindestens Kohlenstoff und/oder Sauerstoff. Gleichfalls können Silicium enthaltende Partikel verwendet werden, die im Wesentlichen bestehen aus amorphen, reinen Silicium Partikel oder auch amorphen, reinen Siliciumcarbid, Siliciumnitrid, der Silicium-Germanium Partikeln vorgenannter Primärpartikelgröße oder Gemischen dieser.

Erfindungsgemäß wird ein großtechnisches, industrielles, vorzugsweise kontinuierliches Verfahren zur Herstellung von Silicium enthaltenden Partikeln, umfassend Primärpartikel und optional Cluster der Primärpartikel bereitgestellt.

Die Umsetzung, insbesondere umfassend die Zersetzung und Bildung der Partikel, kann bei Temperaturen ab 150 °C, bevorzugt ab 400 bis 1500 °C zur Herstellung amorpher Pulver erfolgen. Zur Herstellung amorpher Partikel werden kurze Kontaktzeiten, vorzugsweise bei Temperaturen unter 1300 °C, gewählt. Alternativ kann die Bildung amorpher Primärpartikel bei Temperaturen um 1300 °C bevorzugt kleiner gleich 1100 °C erfolgen. Die Abscheidung der Partikel erfolgt in einer kühleren Zone des Reaktors. Bevorzugte Kontaktzeiten liegen von 10 bis 600 Millisekunden.

So benötigen übliche konventionelle Verfahren, die auf einer Umsetzung von: SiCl4 basieren 30 kW/kg und mehr. Die erfindungsgemäßen Verfahren beruhen vorzugsweise auf einer Umsetzung von Monosilan mit einem Energiebedarf von weniger als 10 kW/kg besonders bevorzugt um 5 kWh/kg. Des Weiteren wird für die Umsetzung im (kalten) Plasma der Energiebedarf weiter reduziert auf kleiner 4 kW/kg.

Als besonders bevorzugt gelten Silicium enthaltende Partikel, in denen weniger als 20 % der Partikel eine Abweichung von der mittleren Korngröße $d_{50}$ und weniger als 5 % eine Abweichung von größer oder gleich 50 % von der mittleren Korngröße $d_{50}$ aufweisen.

Bevorzugt sind weiterhin Silicium enthaltenden Partikel deren Primärpartikel einen mittleren Durchmesser $d_{50}$ (gemessen durch TEM-Auswertung; TEM = Transmissions-Elektronenmikroskop) im Bereich von 5 bis 80 nm, vorzugsweise von 20 bis 50 nm, aufweisen und, die vorzugsweise als aneinandergewachsene Cluster vorliegen. Die Cluster können auch als Agglomerate bezeichnet werden, wobei vorliegend unter einem Cluster aneinandergewachsene bzw. aneinander geschmolzene Primärpartikel verstanden werden. So können die Primärpartikel Cluster ausbilden, in denen mindestens zwei Primärpartikel an ihren Oberflächen aneinander geschmolzen sind. Diese Cluster können als lineare Ketten, in Form von Drähten oder auch verzweigt vorliegen.

Ob die Teilchen sich sphärisch oder in der Form von Whiskern ausbilden, hängt unter anderem von der $H_2$-Konzentration bei der Darstellung ab. Je nach Temperaturprofil, Reinheit (Anwesenheit von metallischen Elementen beispielsweise Eisen - Fe im Gasstrom), Verdünnungsgas (Konzentration, Strömungsgeschwindigkeit) Herstellbedingungen können Primärpartikel isoliert werden oder überwiegend Primärpartikel, die zu Clustern agglomeriert sind, erhalten werden. So können bei einer verdünnten Prozessführung überwiegend Primärpartikel isoliert werden und bei hoher Prozessgaskonzentration und/oder hoher Temperatur, beispielsweise bei 1500°C, Cluster isoliert werden.

Gegenstand der Erfindung ist auch die Verwendung von Clustern der Primärpartikel als Schutz vor elektromagnetischer Strahlung. Dies schließt neben dem UV-VIS-IR das daran anschließende Terraherz-, Hoch- und Höchstfrequenzgebiet sowie den Bereich der Radiowellen bis hin zu Langwellen mit Frequenzen oberhalb 1 Herz mit ein.

Entsprechend einer weiteren Alternative umfassen die Silicium enthaltenden Partikel vorzugsweise Primärpartikel, die im Wesentlichen sphärisch sind. Die mittlere *Sphärizität,* definiert als Aspektverhältnis der Durchmesser der im 90° Winkel aufeinander stehenden Durchmesser, ist vorzugsweise kleiner oder gleich 1,6, vorzugsweise kleiner oder gleich 1,4 bis größer oder gleich 0,9, vorzugsweise von 0,95 bis 1,2. Das Aspektverhältnis nahe der Kugelform erlaubt eine ideale Korrelation der UV Absorption an die Primärpartikelgröße sowie eine gute Homogenisierbarkeit.

Ferner können die Silicium enthaltenden Partikel Primärpartikel und Cluster der Primärpartikel umfassen, insbesondere weisen die Cluster eine Größe von 10 nm bis 3 $\mu$m auf, vorzugsweise von 100 nm bis 3 $\mu$m, weiter bevorzugt von 1 $\mu$m bis zu 6 $\mu$m. Bevorzugte Silicium enthaltenden Partikel umfassen Silicium (Si) oder ggf. SiC, SiGe, SiN-Verbindungen, von größer gleich 99,9999 Gew.-% an Silicium. Optional weisen die SiC, SiGe, SiN-Verbindung eine entsprechenden Gehalt an diesen Verbindungselementen auf. Bevorzugt liegt der Silicium- Gehalt bei größer gleich 94,99 Gew.-%, besonders bevorzugt bei größer gleich 97,999 Gew.-%. Ebenso bevorzugt kann der Silicium-Gehalt bei 98,99 bis größer gleich 99,98 Gew.-% liegen. Ebenso bevorzugt sind Partikel umfassend Silicium-Stickstoff-, Silicium-Kohlenstoff- und/oder Silicium-Germanium-Verbindungen, wie Siliciumnitrid, Siliciumcarbid, Siliciumcarbid in einer Siliciummatrix. Die Silicium-Stickstoff-, Silicium-Germanium-, Silicium-Kohlenstoff-Verbindungen können auch partikulär in einer Matrix von im Wesentlichen reinem Silicium vorliegen. Vorzugsweise liegen die Silicium enthaltenden Partikel im Wesentlichen ohne eine äußere Matrix oder Beschichtung vor, die auch eine passivierende Oxidschicht umfassen kann. Nach einer Alternative liegen die Silicium enthaltenden Partikel mit einer Siliciumdioxid-Zone, dem sogenannten *Core-Shell,* von typisch 1 nm vor. Bevorzugt bzw. im Idealfall umfaßt die Zone eine Monolage auf der Oberfläche.

Dabei ist es erfindungsgemäß besonders bevorzugt, wenn die Silicium enthaltenden Partikel hochrein sind, insbesondere höchstrein. Als hochrein gelten die Partikel, wenn Silicium mit einem Gehalt größer gleich 99,99 Gew.-% in der Gesamtzusammensetzung vorliegt, bevorzugt mit einem Gehalt von größer gleich 99,999 Gew.-%. Als höchstrein gelten die Silicium enthaltenden Partikel mit einem Gehalt von größer gleich 99,9999 Gew.-% Silicium in der Gesamtzusammensetzung, wobei eine Siliciumdioxid Core-Shell außer Betracht bleibt.

Die Verunreinigungen in den jeweiligen Edukten und Verfahrensprodukten werden mittels dem Fachmann bekannter Probenaufschlussverfahren beispielsweise durch Nachweis im ICP-MS (*Analytik für die Bestimmung der Spurenverunreinigung*) bestimmt.

In den erfindungsgemäßen Partikeln weisen die Primärpartikel und optional die Cluster abhängig von ihrer Größe in der Gesamtzusammensetzung einen Gehalt von kleiner oder gleich 2 Atom-%/cm$^3$, vorzugsweise kleiner oder gleich 2000 ppm (Gew.) Sauerstoff auf, vorzugweise kleiner gleich 1000 ppm (Gew.), weiterhin bevorzugt kleiner als 10 ppm (Gew.).

Die Analyse erfolgt neben ICP-MS und HP-GD-MS (*High Purity Glow Discharge Mass Spectroskopy*) oder bevorzugt mittels Neutronenaktivierungsanalyse (NAA). Die NAA ist eine hochempfindliche physikalische Technik der analytischen Chemie zur qualitativen und quantitativen Spurenanalyse, bei der die zu untersuchende Probe mit Neutronen oder anderen Neutralteilchen beschossen wird.

Bei der NAA wird eine Probe von nur wenigen Milligramm bis fallweise zu wenigen $\mu$g dem Neutronenstrom, beispielsweise aus einem Kernreaktor, ausgesetzt. Die Neutronen reagieren mit den Kernen der Probe und machen aus den stabilen Isotopen radioaktive Isotope, deren Massenzahl eins höher ist als die Massenzahl des stabilen Isotops. Bei diesem ersten Kernprozess wird ein promptes $\gamma$-Quant ausgesandt, dessen Energie man messen kann und das Auskunft über den Ausgangskern gibt. In den meisten Untersuchungen wird aber erst der Zerfall des entstandenen radioaktiven Kerns zur Analyse verwendet. Es zerfällt anschließend mit der für ihn typischen Halbwertzeit unter Aussendung eines Betateilchens und charakteristischer Gammastrahlung, die in einem Gammaspektrometer untersucht wird. Auf diese Weise sind praktisch alle vorkommenden Elemente in einer Probe quantitativ und qualitativ nachweisbar. Aus den bekannten Eigenschaften der Atomkerne lassen sich neben dem Gehalt der Elemente sogar ihre Isotope bestimmen. Die Messungen können beispielsweise am *Berlin Neutron Scattering Center* (BENSC) durchgeführt werden.

Der Gehalt an Verdünnungsgasen, wie Xenon, Argon, Krypton, oder auch Stickstoff beträgt in der Gesamtzusammensetzung der Partikel weniger 1% Atome/cm$^3$, insbesondere kleiner gleich 1000 ppm (Gew.), 10 ppm (Gew.), 1 ppb (Gew.) bis zur Nachweisgrenze, beispielsweise bis 1 ppt (Gew.)

Die Nachweisgrenzen für die Bestimmung von Xenon (Xe), Argon (Ar), Krypton (Kr), oder auch Stickstoff durch Neutronenaktivierung bei einer Bestrahlzeit von 1 Stunde bei einer sogenannten Flussdichte an thermischen Neutronen von 10$^{14}$ cm$^{-2}$ s$^{-1}$ liegen bei ca. 10$^{-8}$ g (Ne, Xe) ca. 10$^{-9}$ g (Kr) ca. 10$^{-10}$ g (Ar) und ca. 10$^{-5}$ g für Sauerstoff (O$_2$) Die Nachweisgrenze kann damit insbesondere für kleine Probenmengen kleiner gleich 1000 ppm (Gew.), 10 ppm (Gew.), 1 ppb-Gew bzw. bis zur Nachweisgrenze, beispielsweise bis 1 ppt (Gew.) betragen.

Zur Herstellung der hoch- oder höchstreinen Silicium enthaltenden Partikeln wird ein hoch- bis höchstreines Silan, wobei die Definition von rein bis höchstreines Silane mit Halbleiterqualität verwendet werden, wie monomeres und/oder polymeres Monosilan, H-Silan und/oder Chlorsilan eingesetzt, insbesondere der allgemeinen Formel I, II und/oder III oder eine Mischung der enthaltend, wie höchstreines Tetrachlorsilan, höchstreines Trichlorsilan und/oder höchstreines Dichlorsilan, vorzugsweise mit einem Gehalt an einem Silan von 80 bis 99,9999999 Gew.-%, ad 100 Gew.-% ggf. Polysilane, und mit einer Gesamtverunreinigung von kleiner gleich 100 ppm (Gew.) bis 0,001 ppt (Gew.) als hochreines Silan, vorzugsweise kleiner 50 ppm (Gew.) bis 0,001 ppt (Gew.) als höchstreines Silan, bevorzugt kleiner gleich 40 ppm (Gew.) bis 0,001 ppt (Gew.) an Gesamtverunreinigung mit den nachstehend genannten Elementen. Alternativ kann statt einem einzelnen Silan auch eine Mischung von Silanen eingesetzt werden, sofern sie dem vorgenannten Anforderungsprofil an den Gehalt des Silans entspricht.

Gegenstand der Erfindung ist auch die Verwendung von Silicium enthaltenden Partikeln umfassend

a) Primärpartikel einer Primärpartikelgröße von 1 nm bis 500 nm, insbesondere weisen sie Primärpartikel von 3 bis 100 nm, insbesondere 5 bis 500 nm, 5 bis 100nm, vorzugsweise von 5 bis 80 nm auf. Erhalten werden diese Primärpartikel-größen vorzugsweise über ein Plasma-Verfahren. Des Weiteren ist es bevorzugt, wenn die Partikel zugleich

b) eine mittlere Primärpartikelgröße von im Mittel $d_{50}$ = 16 nm, 20 nm, 25 m, 30 nm oder 40 nm aufweisen, insbesondere jeweils unabhängig mit einer geringen Streuung von +/- 5 nm für $d_{90}$, wobei mindestens ein Anteil der Primärpartikel vorzugsweise als Cluster vorliegt. Alternativ weisen Primärpartikel eine Partikelgröße von 10 bis 50 nm +/- 35 nm auf, insbesondere Primärpartikel von 10 bis 50 nm +/- 25 nm. Primärpartikel, die über ein FSR Verfahren zugänglich sind, weisen in der Regel Primärpartikel mit Partikelgrößen von 100 bis 350 nm auf und vorzugsweise Cluster der Primärpartikel.

Vorzugsweise weisen die erfindungsgemäßen Cluster eine Größe von 10 nm bis 3 $\mu$m auf, weiterhin bevorzugt von 100 nm bis 3 $\mu$m, weiterhin bevorzugt von 200 nm bis 3 $\mu$m.

Dabei ist es bevorzugt, wenn größer oder gleich 70 Gew.-% der Primärpartikel als Cluster vorliegen, vorzugsweise größer 80 Gew.-%, bevorzugt von 85 Gew.-% bis 100 Gew.-%, weiterhin bevorzugt größer als 90, 95, 98, 99,5 Gew.-%.

Je nach Primärpartikelgröße weisen die Silicium enthaltenden Partikel eine hellgelbe, orange oder hellbraune Farbe auf und können daher bei Bedarf auch als Pigmente in kosmetischen Produkten eingesetzt werden. Hier zeigt sich die besonders überraschende Eigenschaft, nämlich zugleich der UV-Schutz und die hautähnliche Farbe. Durch die angenehme hautähnliche warme Eigenfarbe werden die Silicium enthaltenden Partikel, aufgetragen auf die Haut und/oder die Lippen, nicht als störend und unangenehm empfunden, wie es bei bekannten weißen UV-Filtern der Fall ist.

Die erfindungsgemäßen Silicium haltigen Partikel können ferner eine BET-Oberfläche von mehr als 10 m$^2$/g, bevorzugt größer oder gleich 100 m$^2$/g aufweisen.

Entsprechend einer besonders bevorzugten Ausführungsform werden Silicium enthaltende Partikel verwendet, die insbesondere amorph sind, wobei die Partikel die nachfolgende Absorptionscharakteristika elektromagnetischer Strahlung aufweisen:

a) Mit einer Primärpartikelgröße von 10 bis 50 nm, insbesondere mit einer mittleren Partikelgröße von $d_{50}$ um 35 nm, ist die Absorption im Wellenlängenbereich von 250 bis 400 nm im Vergleich zur Absorption im Wellenlängenbereich von 400 bis 750 nm mit einer Abweichung von +/- 40 %, insbesondere +/- 30 %, vorzugsweise +/- 20%, bevorzugt +/-10%, und/oder

b) mit einer Primärpartikelgröße von 10 bis 50 nm, insbesondere mit einer mittleren Partikelgröße von $d_{50}$ um 25 nm, ist das Verhältnis der Absorption im Wellen-längenbereich von (i) 250 bis 400 im Vergleich zur Absorption bei einer Wellen-länge von (ii) 550 nm etwa (i) zu (ii) von 2 : 1 bis 8 : 1, insbesondere 2 : 1 bis 6 : 1, vorzugsweise 3: 1 bis 6 : 1, mit einer Abweichung von +/- 40 %, vorzugsweise +/- 30 %, bevorzugt +/- 20 %, besonders bevorzugt von +/-10%, und/oder

c) mit einer Primärpartikelgröße von 10 bis 50 nm, insbesondere mit einer mittleren Partikelgröße von $d_{50}$ um 25 nm, ist das Verhältnis der Absorption im Wellenlängenbereich von (i) 250 bis 400 im Vergleich zur Absorption bei einer Wellenlänge von (ii) 500 nm bei etwa (i) zu (ii) von 1,5 : 1 bis 8 : 1, insbesondere 1,8 : 1 bis 5 : 1, bevorzugt von 2 : 1 bis 4 : 1, mit einer Abweichung von +/- 30 %, insbesondere bevorzugt +/- 20%, besonders bevorzugt +/-10%, und/oder

d) mit einer Primärpartikelgröße von 10 bis 50 nm, insbesondere mit einer mittleren Partikelgröße von $d_{50}$ um 25 nm, ist das Verhältnis der Absorption im Wellenlängenbereich von (i) 250 bis 350 nm im Vergleich zur Absorption bei einer Wellenlänge von (ii) 450 nm etwa (i) zu (ii) von 2 : 1 bis 4 : 1 mit einer Abweichung von insbesondere +/- 30 %, bevorzugt +/- 20%, besonders bevorzugt +/-10%, und/oder

e) mit einer Primärpartikelgröße von 10 bis 50 nm, insbesondere mit einer mittleren Primärpartikelgröße von $d_{50}$ um 25 nm, ist das Verhältnis der Absorption bei einer Wellenlänge von (i) 250 nm im Vergleich zur Absorption bei einer Wellenlänge von (ii) 450 nm bei etwa (i) zu (ii) von 2 : 1 bis 4 : 1, mit einer Abweichung von insbesondere +/- 30 %, bevorzugt +/- 20%, besonders bevorzugt +/-10%, und/oder

f) mit einer Primärpartikelgröße von 10 bis 50 nm, insbesondere mit einer mittleren Partikelgröße von $d_{50}$ um 25 nm, ist das Verhältnis der Absorption bei einer Wellenlänge von (i) 350 nm im Vergleich zur Absorption bei einer Wellenlänge von (ii) 450 nm bei etwa (i) zu (ii) von 2 : 1 bis 3 : 1, mit einer Abweichung von insbesondere +/- 30 %, bevorzugt +/- 20%, besonders bevorzugt +/-10%.

Die unter a) bis f) genannten Werte wurden insbesondere für reine, hochreine bis höchstreine und im wesentlichen amorphe Silicium enthaltende Partikel mit einem Siliciumgehalt von größer oder gleich 98 Gew.-% und optional mit einem Gehalt an Kohlenstoff und/oder Sauerstoff erzielt. Bevorzugt ist ein Gehalt an Silicium von größer gleich 99,5 Gew.-% und optional zusätzlich Sauerstoff, bezogen auf 100 Gew.-% in der Gesamtzusammensetzung. Vorzugsweise werden die jeweilig angegebenen Absorptionen über den Wellenlängenbereich gemittelt.

Gegenstand der Erfindung ist gleichfalls die Verwendung von Silicium enthaltenden Partikeln, insbesondere amorphen und im Wesentlichen Silicium enthaltenden Partikeln einer Primärpartikelgröße von 1 bis 100 nm zur Erhöhung der Brechzahl einer kosmetischen, dentalen, medizinischen oder pharmazeutischen Formulierung. Als medizinische Formulierungen ist ein Sprühverband oder eine Wundauflage denkbar.

Ebenfalls Gegenstand der Erfindung ist die Verwendung von Silicium enthaltenden Partikel ausgewählt aus Partikeln mit einem Gehalt an reinem Silicium und SiC mit Silicium 70 bis 90 Gew.-% und 10 bis 30 Gew.-% Kohlenstoff sowie reinem SiC zur Erhöhung der Brechzahl einer Formulierung oder eines Materials. Vorzugsweise weisen die Silicium enthaltenden Partikel zu 60 bis 100 Gew.-% Silicium auf und auf 100 Gew.-%, beispielsweise zu 0 bis 40 Gew.-%, weisen sie einen Gehalt an Kohlenstoff und optional Sauerstoff auf, vorzugsweise sind die Partikel transparent. Weiter bevorzugt umfassen die Siliciumpartikel zu 70 Gew.-% Silicium und auf 100 Gew.-% einen Gehalt an Kohlenstoff und optional Sauerstoff. Dabei können Silicium enthaltende Partikel, insbesondere ausgewählt aus Partikeln mit einem Gehalt an Silicium größer 90 bis 100 Gew.-%, vorzugsweise größer gleich 91 bis 100 Gew.-%, vorzugsweises größer gleich 95, 98, 99,5 bis 100 Gew.-%, eine Brechzahl (n) von größer gleich 3,0 bei einer Wellenlänge von 500 bis 2500 nm, und/oder eine Brechzahl (n) von größer gleich 4,0 bei einer Wellenlänge von 200 bis 500 nm aufweisen, insbesondere von 280 bis 400 nm. Bevorzugt weisen die Partikel, insbesondere reine und amorphe Silicium Partikel eine Brechzahl (n) von größer gleich 5 bis 7 im Wellenlängenbereich von etwa 280 bis 400 nm auf, besonders bevorzugt von 6 bis 7. Vorzugsweise weisen im Wesentlichen reine, amorphe Silicium Partikel mit einer Primärpartikelgröße von im Mittel 20 bis 40 nm diese Brechzahlen auf. Die Brechzahlen erlauben somit auch eine Qualitätskontrolle der Reinheit der hergestellten Silicium enthaltenden Partikel.

Gleichfalls Gegenstand der Erfindung ist die Verwendung von Silicium enthaltenden Partikel, insbesondere ausgewählt aus Partikeln mit einem Gehalt an Silicium von größer gleich 50 Gew.-% bis 100 Gew.-%, die optional 0 bis 50 Gew.-% Kohlenstoff umfassen, vorzugsweise 0,001 bis 30 Gew.-%, bevorzugt 0,001 bis 20 Gew.-%, besonders bevorzugt 5 bis 15 Gew.-% Kohlenstoff, vorzugsweise beträgt der Gehalt an Kohlenstoff um 10 Gew.-% Kohlenstoff mit einer Schwankungsbreite von +/- 3 Gew.-%. Zusätzlich oder alternativ können die Silicium enthaltenden Partikel 0 bis 20 Gew.-% Sauerstoff aufweisen. Vorzugsweise weist die äußere Beschichtung, gleichbedeutend mit der Core-Shell, einen Gehalt an Sauerstoff auf. Vorzugsweise beträgt der Gehalt an Sauerstoff von 0,0001 bis 10 Gew.-%, weiter bevorzugt von 0,0001 bis 5 Gew.-5. Dabei ist es bevorzugt, wenn die mittlere Primärpartikelgröße 20 bis 40 nm beträgt und/oder die Primärpartikel eine Größe von 10 bis 80 nm aufweisen.

Weiter ist es bevorzugt, wenn die Partikel eine Core-Shell mit einem Gehalt an Sauerstoff aufweisen, wobei die Partikel in diesem Fall vorzugsweise einen Gehalt an Si-O und/oder Si-OH Gruppen aufweisen. Partikel mit einer Core-Shell, die auch definiert im Verfahren hergestellt werden können, sind beispielsweise einer Silanisierung und damit einer weiteren Modifizierung und Anbindung oder Einbindung in anderen Materialien zugänglich.

Ein weiterer Vorteil der erfindungsgemäßen Partikel ist ihre Verwendbarkeit als anorganischer UV-Schutz und vorzugsweise Schutz vor Kratzern, beispielsweise im Bereich polymerer oder anorganischer Oberflächen. Die Anwendung als UV- und Kratzschutz ist dabei nicht auf eine unmittelbare Verwendung zum Schutz von Zellen beschränkt, sondern kann auch mittelbar zur Schutz von Zellen beispielsweise auf Sonnenbrillen und dergleichen mehr zum Einsatz kommen und mittelbar dem Schutz von Zellen dienen.

Des Weiteren können nach einer Alternative die Silicium enthaltenden Partikel, die als Primärpartikel und optional Cluster der Primärpartikel vorliegen, und ggf. Siliciumcarbid, Siliciumnitrid, Silicium-Germanium enthalten, zusätzlich mit einem Elektronenakzeptor oder Elektronendonator dotiert werden. Zur Dotierung wird während der Herstellung mindestens ein unter den Reaktionsbedingungen reaktives Dotiergas zugegeben. Bevorzugt ist beispielsweise Diboran. Ebenso kann ein Selen enthaltendes Gas zugesetzte werden.

Nach einer besonders bevorzugten Alternative ist Gegenstand der Erfindung die Verwendung von Silicium enthaltenden Partikeln umfassend Primärpartikel von 1 bis 100 nm, vorzugsweise 5 bis 100 nm, und optional Cluster dieser Primärpartikel, mit einem Gehalt an Silicium von größer gleich 90 Gew.-% bis 100 Gew.-% als biologisch verträglicher UV-Schutz und/oder als biologisch abbaubarer UV-Schutz. Dabei ist es weiter bevorzugt, wenn die Partikel von Zellen, wie den Hautzellen oder Körperzellen abgebaut werden können. So kann der Körper vorzugsweise die amorphen Siliciumpartikel resorbieren und abbauen. Das Siliziumpartikel wird sukzessive zu $SiO_2$ im wässrigen biologischen System umgesetzt. Abhängig vom pH-Wert des Biosystems wird dieses aufgelöst.

Die Partikel können im Wesentlichen als sphärische Partikel oder auch als plättchenförmige Partikel mit einer Schichtdicke von 1 bis 80 nm, insbesondere 20 bis 45 nm, vorliegen. Als plättchenförmige Partikel können die Partikel vorliegen, wenn die Abscheidung der Partikel auf einer kalten Fläche erfolgt oder die Partikel in eine Folie eingebracht werden.

Gegenstand der Erfindung ist ebenfalls ein Verfahren zur Herstellung von Silicium enthaltenden Partikeln, sowie Silicium enthaltende Partikel erhältlich nach diesem Verfahren, indem

    a) mindestens eine gasförmige oder bei erhöhter Temperatur gasförmige Siliciumverbindung,

b) optional in Gegenwart mindestens eines unter den Reaktionsbedingungen reaktiven Gases oder einer Mischung von reaktiven Gasen, insbesondere bei erhöhter Temperatur oder im Plasma reaktiven Gases,

c) in Gegenwart eines Verdünnungsgases in einer im Wesentlichen sauerstofffreien Atmosphäre unter (i) thermischen Bedingungen und/oder (ii) im Plasma zersetzt wird, und

d) mit einem Fluid als Silicium enthaltende Partikel abgeschieden wird.

Die Siliciumverbindung kann eine gasförmige Verbindung sein, wie vorzugsweise Monosilan oder auch eine Verbindung, die bei erhöhter Temperatur und/oder vermindertem Druck in die Gasphase übergeht.

Die Abscheidung als Silicium enthaltende Partikel, insbesondere des gebildeten Feststoffes, kann in einem Fluid beispielsweise durch Wäsche erfolgen, vorzugsweise, indem die Zersetzungsprodukte der Siliciumverbindung und optional des mindestens einen reaktiven Gases mit einem flüssigen oder gasförmigen Fluid, wie Paraffin, $N_2$, Edelgase, $CO_2$ gasförmig oder überkritisches $CO_2$, abgekühlt bis abgeschreckt werden. Bevorzugt können die Zersetzungsprodukte als Silicium enthaltende Partikel mit einem Fluid in Form einer Lösung, Emulsion, Suspension, Gel, Schaums, Aerosols oder Rauch abgeschieden und insbesondere isoliert werden. Die so hergestellten Partikel können je nach Prozessbedingungen als überwiegend isolierte Primärpartikel, die vorzugsweise nicht weiter stabilisiert werden müssen, oder als überwiegend als Cluster von Primärpartikel, die jeweils amorph vorliegen, abgeschieden werden. Eine Stabilisierung kann beispielsweise durch Verwendung sogenannter Ionischer Flüssigkeiten verbessert werden.

Als Fluid kommen weiter beispielsweise in Betracht: natürliches oder synthetisches Öle/Fette, Emulgatoren, Wachse, Kohlenwasserstoffe, wie Paraffinöl, Siliconöl, pflanzliche Fette und Öle, synthetische Glyceride, Kakaobutter, Mandelöl, Erdnussöl, Sheabutter, tierische Wachse (Wollwachs, Bienenwachs), hydrierte Öle (hydriertes Rizinusöl, hydriertes Sojaöl), Emulgatoren, diese entweder vom O/W-Typ (z. B. Polysorbat, Macrogolether, Fettalkoholsulfate) oder vom W/O-Typ (Wollwachsalkohole, Sorbitanfettsäureester, Monoglyceride), (Teil-)oxidierte Kohlenwasserstoffe, Fette/Öle etc. sowie weitere dem Fachmann bekannte übliche und vorzugsweise pharmakologisch verträgliche Fluide.

Als gasförmige Siliciumverbindung kommen generell alle Wasserstoff enthaltenden Silane, wie Monosilan, Disilan, Trisilan und Gemische enthaltend mindestens eines der Silane in Betracht sowie Halogen und Wasserstoff oder rein Halogen enthaltende Silane und Polysilane, die auch im Gemisch in dem Verfahren umgesetzt werden können. Vorzugsweise kann die Siliciumverbindung Spuren von Si-Cl, Si-Br und/oder Halogensilane aufweisen oder eine in Spuren zugesetzte oder vorhandene Si-Cl enthaltende Verbindung. Die bei erhöhter Temperatur gasförmige Siliciumverbindung kann auch Kohlenwasserstoff-enthaltende Silane umfassen. Bevorzugte Silane sind Halogensilane, Chlorsilane, wie Dichlorsilan, Trichlorsilan, Tetrachlorsilan, Hexachlordisilan, Methyltrichlorsilan, Polyhalogensilane sowie reine H-Silane wie Monosilan, Wasserstoff enthaltende Polysilane oder Polyhalogensilane und/oder mindestens ein Alkoxysilan.

Besonders bevorzugte Abkühlungsbedingungen werden nachfolgend näher erläutert. Wie vorstehend ausgeführt, kann die Bildung der Pulver auf zweierlei Weise erfolgen. So kann die Abkühlung und Ausbildung der Silicium enthaltenden Partikel durch Reaktion im kühlen, insbesondere mit flüssigem Stickstoff gekühlten reaktiven Gasstrom, erfolgen. Der reaktive Gasstrom kann Kohlenwasserstoffe enthalten. Nach der Bildung der Silicium enthaltenden Partikel erfolgt die weitere Abkühlung der Zersetzungsprodukte durch Einleiten der gasförmigen Zersetzungsprodukte in flüssiges Fluide, Kühlmittel, beispielsweise flüssiges Helium oder in ein flüssiges reaktives Gas. In diesem Fall bindet sich auf dem Silicium enthaltenden Partikel unmittelbar das reaktive Gas. Damit kann eine Funktionalisierung gegeben sein.

Eine Abkühlung des Prozessgases aus dem heißen, nicht-thermischen Plasma kann auch durch geeignete Prozessführung erfolgen, beispielsweise durch Einleiten in inerte, kühle und leicht verdampfbare Medien oder durch Einleiten in flüssige Siliciumverbindungen und/oder Bor oder Selen Enthaltende. Generell kann die Abscheidung erfolgen, indem die Fluide zur Abscheidung eine Temperatur deutlich unterhalb 1000 °C aufweisen, vorzugsweise unter 200 °C, weiter bevorzugt unterhalb 100 °C, besonders bevorzugt unterhalb 50 °C bis -273 °C. Besonders bevorzugt erfolgt ein rasches Abscheiden durch Einstellen einer Temperaturdifferenz von größer gleich 100 °C, insbesondere größer gleich 200 °C, bevorzugt von größer gleich 500 °C, innerhalb von einer Minute, vorzugsweise innerhalb von 1000 Millisekunden, unterhalb des jeweiligen Schmelzpunktes der Silicium enthaltenden Partikel, besonders bevorzugt innerhalb von kleiner gleich 200 Millisekunden, um im Wesentlichen amorphe Partikel zu erhalten.

Alternativ können die amorphen Primärpartikel erhalten werden in einem im nicht-thermischen Gleichgewicht befindlichen Plasma, dessen Temperaturen bei kleiner gleich 1050 °C, vorzugsweise kleiner gleich 700 °C besonders bevorzugt bei kleiner gleich 150 °C liegen. In einer anderen Variante wird das Verfahren bevorzugt im Tieftemperaturbereich durchgeführt, d. h. im Bereich zwischen 373 Kelvin und größer 0 Kelvin.

Erfindungsgemäß bevorzugt umfasst das Plasma die Bedingungen einer Gasentladung, insbesondere das nicht-thermische Plasma.

Erfindungsgemäß eingesetzte nicht-thermische Plasmen werden beispielsweise durch eine Gasentladung oder durch Einstrahlung von elektromagnetischer Energie, wie durch Einstrahlung von Radio- oder Mikrowellen, in einer Reaktionskammer erzeugt. Die Erzeugung des Plasmas erfolgt also nicht, wie bei thermischen Plasmen, durch hohe Temperaturen, sondern durch nicht-thermische Ionisationsprozesse. Dem Fachmann sind solche Plasmen bekannt.

Beispielhaft sei hierzu der sogenannte Penning-Ionisationsprozess genannt.

Für die vorstehend aufgeführten Verfahren wurden im nicht-thermischen Gleichgewicht betriebene Gasentladungen verwendet. Nicht-thermisch bedeutet im erfinderischen Sinne, dass die Elektronen als energievermittelnde Spezies eine höhere Temperatur und damit eine höhere Energie als die schwereren geladenen oder ungeladenen Teilchen (zum Beispiel N, $N_2$, $N_2^+$, $N^+$, Si, SiH, $SiH_2^+$, C, H, $H_2$, NH) aufweisen. Die dafür erforderlichen Energien können durch dem Fachmann bekannte bzw. geläufige Vorschaltgeräte bzw. Netzteile erzeugt werden. Das nicht-thermische Plasma weist im Allgemeinen Elektronen mit einer Energie im Bereich von 0,1 bis 100 eV auf, vorzugsweise von 1 bis 50 eV. Die schwereren Teilchen weisen eine Energie im Bereich von 0,000 001 bis 10 eV, bevorzugt von 0,01 bis 0,1 eV auf.

Überraschenderweise hat es sich gezeigt, dass eine solche nicht-thermische Gasentladung sich vorteilhaft durch Dimmen mittels Phasenanschnittsteuerung oder/und über Pulsweitenmodulation oder über die Pulsfrequenz erzeugen lässt, wobei vorteilhafterweise die Elektroden als Hohlelektroden mit vorzugsweise porösen Stirnflächen, beispielsweise aus Sintermetall, durch eine zweidimensionale parabolische Form ausgeführt sind. Damit verteilt sich der Gasstrom gleichmäßig über die Elektrodenoberfläche. Die zweidimensionale pilzartige Oberfläche kann gemäß der folgenden Beziehung beschrieben werden:

$$F(r) = r^2 \, , \quad \text{mit } 0{,}1 < r < 1{,}1 \text{ cm}$$

Das erfindungsgemäße Verfahren wird im Allgemeinen in nicht-thermischen Plasmen durchgeführt, welche Temperaturen von 100 bis 3400 °C, vorzugsweise von 700 bis 999 °C aufweisen.

Das Plasma kann gepulst werden. Vorzugsweise wird ein im Wesentlichen zylinderförmiges Plasma, das insbesondere nicht-thermisch ist, in einem zylindrischen Bereich des Reaktionszylinders vorgesehen.

Bei den Synthesen im Plasma kann zweckmäßig mit einem Inertgas, beispielsweise einem Edelgas oder eine Mischung von Edelgasen, z. B. Argon mit geringen Anteilen an Helium, und/oder Krypton, Xenon sowie auch mit reaktiven Gasen wie Stickstoff, Wasserstoff, Methan, Tetrachlorkohlenstoff etc. gearbeitet werden. Andere Gasmischungen sind dem Fachmann bekannt oder können einschlägigen Lehrbüchern entnommen werden.

Eine weitere bevorzugte Ausführungsform des erfindungsgemäßen Verfahrens beinhaltet das Einleiten von Edelgas oder von Edelgasgemischen oder von Edelgas-Gasgemischen aus der Kombination Argon und/oder Helium, Krypton, Xenon als Verdünnungsgas oder von Gemischen von Edelgasen und reaktiven Gasen Stickstoff, Wasserstoff, Methan, Tetrachlorkohlenstoff etc. in das nicht-thermische Plasma, das insbesondere Temperaturen von kleiner gleich 3000 °C, vorzugsweise kleiner gleich 1900 °C aufweist.

In dem Verfahren nach der Erfindung können vorzugsweise weitere bei erhöhter Temperatur gasförmige Siliciumverbindungen eingesetzt werden. Diese umfassn Wasserstoff- und/oder Halogen-enthaltende Silane, wie H-Silane, Halogensilane, und/oder Kohlenwasserstoff-enthaltende Silane, wie Methylsilan, Methyltrichlorsilan, Dimethyldichlorsilan, Halogensilane, Chlorsilane sowie reine, hochreine, insbesondere höchstreine H-Silane, wie Monosilan und/oder Chlorsilane, wie Dichlorsilan, Trichlorsilan, Tetrachlorsilan, Hexachlordisilan, Methyltrichlorsilan, Polyhalogensilane, Wasserstoff enthaltende Polysilane, umfassend ausschließlich Wasserstoff oder Wasserstoff und Halogen. Zur Herstellung der amorphen Partikel können gleichfalls Alkoxysilane, vorzugsweise Tetramethoxysilan, Tetraethoxysilan oder gemischte Tetraalkoxysilane eingesetzt werden.

Erfindungsgemäß einsetzbare Silane umfassen Silane der allgemeinen Formel **I**,

I $\qquad H_x SiCl_{4-x}$,

mit x unabhängig voneinander ausgewählt aus 0, 1, 2 oder 3. Vorzugsweise ist x gleich 0, 1 oder 2, bevorzugt ist x gleich 0 oder 1. Besonders bevorzugt ist x gleich 0, oder ein Gemisch umfassend mindestens zwei monomere Chlorsilane der Formel I, ausgewählt aus Tetrachlorsilan, Trichlorsilan und Dichlorsilan, bevorzugt reines Tetrachlorsilan oder reines Tetrachlorsilan mit einem Gehalt an Trichlorsilan und/oder Dichlorsilan.

Bevorzugt können auch Polyperchlorsilangemische eingesetzt werden, die Polyperchlorsilane mit 2 bis 8 Siliciumatomen umfassen. Polychlorsilane bis 6 Siliziumatome sind einfach verdampfbar, wogegen Verbindungen ab 7 Siliciumatomen als Aerosol prozessiert werden. Besonders bevorzugt ist auch die Verwendung höhermolekularer Polychlorsilane mit mindestens drei Siliciumatomen, insbesondere mit 3 bis 8 Siliciumatomen.

Bevorzugt sind Polyperchlorsilangemische umfassend Polyperchlorsilane mit 2 bis 100 Siliciumatomen. Besonders bevorzugt ist auch die Verwendung höhermolekularer Polychlorsilane mit mindestens drei Siliciumatomen, bevorzugt mit 3 bis 50 Siliciumatomen, die durch komproportionierende Reaktion $[SiCl]_n$ dargestellt werden können, wobei das $[SiCl]_n$-Molekül bevorzugt als Sechserringnetz, gleichbedeutend mit n = 6, sowie dessen Vielfache vorliegen. Die Analyse erfolgt im infraroten Spektralbereich oder durch Si29-NMR.

Die Verwendung von Polychlorsilane entsprechend der Erfindung umfassen die homologe Reihe der Polyperchlorsilane der allgemeinen Formel II,

$$II \qquad Si_nCl_{2n+2},$$

mit $n \geq 2$, welche lineare und/oder verzweigte Ketten bilden, sowie die Polyperchlorsilane, die Ringe oder Polymere bilden, wobei die Polymere auch verzweigt und/oder cyclisch sein können, und ebenfalls mit der idealisierten Formel III,

$$III \qquad Si_nCl_{2n},$$

mit $n \geq 3$, als auch die Siliciumchloride mit geringerem Chlorgehalt der idealisierten Formel IV:

$$IV \qquad SiCl_{1,5}.$$

Besonders bevorzugt gelten als Polychlorsilane Verbindungen der allgemeinen Formel II mit $n \geq 2$, bevorzugt mit $2 \leq n \leq 100$, weiter bevorzugt mit $2 \leq n \leq 50$, vorzugsweise jeweils unabhängig mit n grösser oder gleich 2, 3, 4, 5, 6, 7, 8, 9 oder 10, bevorzugt 2 bis 8, besonders bevorzugt mit n gleich 2 oder 3, wobei die Verbindungen lineare als auch verzweigte Ketten bilden können. Ebenfalls sind Verbindungen der allgemeinen Formel III bevorzugt, die Ringe oder Polymere bilden mit $Si_nCl_{2n}$, mit $n \geq 3$, insbesondere mit $4 \leq n \leq 100$, vorzugsweise $4 \leq n \leq 50$, besonders bevorzugt jeweils unabhängig mit $n \geq 4$, $n \geq 5$, $n \geq 6$, $n \geq 7$, $n \geq 8$, $n \geq 9$ oder $n \geq 10$, als auch Polychlorsilane mit geringerem Chlorgehalt gemäß der allgemeinen Formel IV mit $SinCl_{1,5n}$, mit $n \geq 4$ oder $n \geq 5$, insbesondere mit $6 \leq n \leq 200$, vorzugsweise mit $8 \leq n \leq 100$.

Besonders bevorzugt wird ein Polychlorsilan (PCS) eingesetzt, insbesondere Octachlortrisilan, oder ein Octachlortrisilan in einer Mischung mit höhermolekularen Polychlorsilanen, vorzugsweise Polyperchlorsilanen, wobei das Polychlorsilan einen Gehalt an Octachlortrisilan von 0,20 bis 99,9999 Gew.-%, vorzugsweise von 10 bis 99,9999 Gew.-% aufweist. Diese Silane können die oben genannten Verunreinigungen aufweisen.

Ebenfalls kann eingesetzt werden ein gelöstes Polysilan und/oder Polychlorsilan, beispielsweise in einem reaktiven Lösemittel, z.B. Kohlenwasserstoff als Flüssigkeit, Sirup, Paste, Creme, Dispersion, Emulsion, wobei das hochreine Lösemittel vor der Zersetzung zu einem reaktiven Gas verdampft wird. Im Rahmen der Erfindung gelten auch reaktive verdampfbare Lösemittel als reaktive Gase.

Vorzugsweise wird in dem Verfahren ein inertes Verdünnungsgas verwendet. Je nach Anforderungsprofil kann auf das Verdünnungsgas verzichtet werden, wenn zugleich ein Vakuum anliegt. Bevorzugte Verdünnungsgase sind Argon, Helium, Xenon, Krypton oder ein Gemisch umfassend mindestens zwei der genannten Gase. Stickstoff ist unter den genannten Bedingungen in dem Plasma oder nicht-thermischen Plasma kein inertes Verdünnungsgas, sondern ein reaktives Gas. Das Verdünnungsgas kann vorzugsweise mit der gasförmigen Silicium-Verbindung und/oder dem reaktiven Gas vor dem Einbringen in den Reaktionszylinder gemischt werden. Alternativ wird das inerte Verdünnungsgas zur Abscheidung der Partikel verwendet werden. Die Zersetzung der Silicium-Verbindung und optional des reaktiven Gases kann auch durch Überführen der in einen Hochvakuum-Bereich erfolgen. Als ein Hoch-Vakuum wird ein Vakuum kleiner gleich 0,01 bar, insbesondere kleiner gleich 0,001 bar, kleiner gleich 0,0001 bar erachtet.

Der Druckbereich liegt üblicherweise bei 0,001 mbar bis 50 bar, bevorzugt bei 1 mbar bis 10 bar, weiter bevorzugt bei 10 mbar bis 5 bar. Je nach dem gewünschten Zersetzungs- und/oder Legierungs- und/oder Beschichtungsprodukt kann das Verfahren auch in einem Druckbereich von 1 bis 50 bar erfolgen, bevorzugt bei 2 bis 50 bar, besonders bevorzugt bei 5 bis 50 bar. Damit wird die Bildung von Kohlenstoff enthaltenden Prozessgasen minimiert. Dabei weiß der Fachmann, dass der zu wählende Druck ein Kompromiss zwischen Gasabführung, Agglomerierung und Reduktion der Kohlenstoff enthaltenden Prozessgase ist.

Bevorzugt einsetzbare reaktive Gase umfassen Stickstoff enthaltende Verbindungen, Germanium enthaltende Verbindungen, Wasserstoff, Stickstoff ($N_2$), $NH_3$, Hydrazin, Stickstoffwasserstoffsäure, Kohlenwasserstoffe, wie Methan, Butan, Propan, höchstreines Erdgas, aromatische Verbindungen, wie Toluol, insbesondere jeweils sauerstofffreie Verbindungen, mit der Maßgabe, dass sich bei der Zersetzung kein Wasser bildet. Bevorzugte reaktive Gase umfassen Kohlenwasserstoffe, wie Methan, Ethan, Propan, Butan, Gemische dieser Gase, weiterhin HCl, und/oder Kohlenwasserstoffe mit Stickstoff.

Zur Durchführung des Verfahrens ist es zusätzlich zu den vorgenannten Merkmalen weiter bevorzugt, wenn die Gasentladung ein nicht-thermisches Plasma ist. Weiter bevorzugt erfolgt die Anregung der Gasentladung durch einen Generator, der auch in einem Ozonisator Anwendung findet. Für die Definition des nicht-thermischen Plasmas wird auf die einschlägige Fachliteratur verwiesen, wie beispielsweise auf "Plasmatechnik: Grundlagen und Anwendungen - Eine Einführung; Autorenkollektiv, Carl Hanser Verlag, München/Wien; 1984, ISBN 3 446-13627-4".

Der spezifische Leistungseintrag beträgt von 0,001 und 1000 W/cm$^2$.

Besonders bevorzugt beträgt der spezifische Energieeintrag von 0,1 und 100 Ws/cm$^2$. In diesen Fällen kann die Spaltweite (GAP) 1 mm betragen. Dabei ist es weiter bevorzugt, wenn der spezifische Energieeintrag mittels phasengenauer Momentanleistungsmessung mit einer Bandbreite von mindestens 250 kHz durchgeführt wird. Die Bestimmung der Momentanleistung erfolgt in einer normierten Anordnung mit 50 cm$^2$ Entladungsfläche. Der Energieeintrag zur Ausbildung des nicht-thermischen Plasmas erfolgt vorzugsweise in der Art, dass sich in dem sich ausbildenden Plasma möglichst homogene Bedingungen für die Umsetzung der Silane sowie der C, N und/oder Ge oder dergleichen mehr enthaltenen Verbindungen ausbilden. Dabei ist es besonders bevorzugt, wenn das nicht-thermische Plasma bei einer Spannung betrieben wird, bei der die Entladung die gesamte Elektrodenfläche bedeckt.

Entsprechend einer bevorzugten Alternative werden die abgeschiedenen Partikel an oberflächlich vorhandenen Sauerstoff-Atomen und/oder Chlor-Atomen organofunktionalisiert. Vorzugsweise werden die Partikel an der Oberfläche der Partikel durch Reaktion mit einer reaktiven organofunktionellen Gruppe des Silans modifiziert. Die Modifizierung kann über Komplexierung oder eine Ausbildung kovalenter Bindungen erfolgen. Generell können die Silicium enthaltenden Partikel zumindest teilweise mit einem organofunktionellen Silan modifiziert werden. Organofunktionelle Silane umfassen Silane mit ungesättigten Kohlenwasserstoffresten, Halogenfunktionalisierte Silane, wie vorzugsweise Halogenalkylsilane, wie Monochlortrimethylsilan, Halogenalkoxysilane, wie Monochlortrialkoxysilan, Alkylenalkoxysilane, Alkylenhalogensilane, Amino-funktionelle Silane, wie Aminopropyltriethoxysilan, Aminopropyltrialkylsilan, sowie organofunktionelle Silane. Organofunktionelle Silane umfassen auch organisch funktionalisierte Siliciumverbindungen und Organosiloxane.

Gegenstand der Erfindung sind auch Formulierungen enthaltend die Silicium enthaltenden Partikel, wobei diese Hilfsmittel, Lösungsmittel, Emulgator und/oder Dispersionsmittel und/oder Suspensionsmittel enthalten können. Besonders bevorzugte Formulierungen enthaltend amorphe Silicium enthaltende Partikel mit einen Gehalt von 50 bis 100 Gew.-% Silicium, vorzugsweise mit reinen Siliciumpartikeln mit einem Gehalt an Silicium größer oder gleich 99,95 Gew.-%, besonders bevorzugt größer oder gleich 99,9999 Gew.-%, und einer mittleren Primärpartikelgröße $d_{50}$ von 5 bis 500 nm. Bevorzugt ist $d_{50}$ von 5 bis 40 nm, wobei die Partikel vorzugsweise als Cluster von Primärpartikeln vorliegen.

Gegenstand der Erfindung ist auch eine Formulierung enthaltend Silicium enthaltende Partikel als eine kosmetische, medizinische oder pharmazeutische Formulierung für den UV-Schutz der Haut oder der Haare, insbesondere eine tropische Sonnenschutzformulierung, zum Beispiel eine Sonnenschutzcreme, ein Sonnenschutzgel, Sonnenschutzemulsion, -öl oder eine sprühbare Sonnenschutzformulierung. Auch Wundauflagen, Augentropfen sind mögliche Formulierungen. Die erfindungsgemäße Formulierung umfasst vorzugsweise mindestens ein Hilfsmittel wie Bisabolol, Wollwachse (Lanolin), Siliciumdioxid wie zum Beispiel Aerosil®, Bentonit, Glycerol, Celluloseether, wie Methylcellulose, Haxdroxyethylzellulose, Ethylhydroxyethylcellulose, Natriumcarboxymethylcellulose, Alginat, Stärke, Tragant, Polyacrylsäuren, Polyvinylalkohol, Polyvinylpyrrolidion, pflanzliche oder synthetische Fette und Öle.

Eine erfindungsgemäße Formulierung umfasst vorzugsweise mindestens ein Hilfsmittel, Additiv oder weitere übliche Formulierungsbestanteile. Vorzugsweise liegt die Formulierung zur tropischen Anwendung vor oder zur Anwendung im Auge, wie als Lösung, Suspension, Emulsion. Besondere Darreichungsformen können umfassen Salben, Cremes, Hydrogele, Gel, Spray, Puder, filmbildendes Spray, Folie sowie weitere dem Fachmann geläufige kosmetische und/oder medizinische Formulierungen und Darreichungsformen.

Die erfindungsgemäßen Partikel eignen sich aufgrund ihrer Inertheit und ihres Transmissionsverhaltens besonders gut als UV-Schutzmittel, da die erfindungsgemäßen Partikel, wie die SiC-Pulver, transparent vorliegen können und vorteilhaft bei 300 nm keine nennenswerte Transmission aufweisen.

Die amorphen Partikel sind vorzugsweise nicht kristallin. Als im Wesentlichen amorph gilt ein Pulver, das röntgenamorph ist. Als röntgenamorph gilt vorzugsweise ein Pulver mit einem Kristallinität kleiner 2 %. Der Kristallinitätsgrad wird mittels XRPD berechnet unter Anwendung der Beziehung:

$$\text{Kristallinität in \%} = (100 \times A)/(A + B - C) .$$

Die Größen A, B, und C bedeuten:

A = gesamte Peakfläche der Reflexe der kristallinen Bestandteile des Diffraktogramms.
B = gesamte Fläche unterhalb der Peakfläche A.
C = luftstreuend-, fluoreszierend- und gerätebedingte Untergrundfläche.

In den Beispielen wurde die Untergrundfläche C anhand der XRD-Diagramme des Si-Referenzstandards NIST 640 (Si-Standard = 100% Kristallinität) ermittelt. Die Fläche B entsprach einem eingelegten Untergrundverlauf sowie dem konstanten Untergrund C. Für die Berechnung wurde die dem Fachmann bekannte *"HighScore Plus Software"* eingesetzt.

In röntgenamorphen Pulvern gibt es im XRPD keine scharfen Linienformen, sondern nur wenige diffuse Interferenzen bei kleinen Beugungswinkeln. Stoffe mit einem derartigen Röntgenbeugungsdiagramm bezeichnet man als *röntgenamorph.* Ein Kristall, gleichbedeutend mit einem anisotropen, homogenen Körper mit einer dreidimensional periodischen Anordnung der Bausteine zeigt im XRPD klar definierte auflösbare Reflexe.

Sowohl die Partikelgrößen, als auch die Bildung von Clustern, z.B. Agglomeraten, und/oder das Vorliegen von Primärpartikeln können neben anderen dem Fachmann bekannten Methoden mittels Siebanalyse, Transelektronenmikroskopie (TEM), Rasterkraftelektronenmikroskopie (REM) oder Lichtmikroskopie nachgewiesen bzw. bestimmt werden.

**Ausführungsbeispiele**

Figur 1a     zeigt eine TEM-Aufnahme der hergestellten Si-Partikel (Si)
Figur 1b     zeigt das UV-Vis-Spektrum des erfindungsgemäßen Produktes Probe 4 (P4), Siliziumpartikel mit 10- 50 nm Größe mit Mittelwert ca. 35nm sowie das erfindungsgemäßes Produkt 3 (P3) Siliziumpartikel mit 10-50 nm Größe mit Mittelwert ca. 25nm.
Figur 2:     Brechzahl von Silicium in Abhängigkeit von der Wellenlänge ($\lambda$), Spektrum e: Brechzahl Silicium, Linie f: Brechzahl n = 4 .
Figur 3a     zeigt die Silicium enthaltenden Partikel nach Ausführungsbeispiel 2a.
Figur 3b     zeigt Silicium enthaltende Partikel nach Ausführungsbeispiel 2b.

Figur 1b zeigt das UV-Vis-Absorptionsverhalten einer Referenzprobe sowie von Silicium unterschiedlicher Primärpartikel- und unterschiedlicher Clustergrößen. In Richtung des Pfeils (Zunahme Partikelgröße) nimmt die Absorption im Wellenlängenbereiche von oberhalb 400 nm bis weit über 1000 nm mit zunehmender Partikelgröße zu. Zuordnung der Spektren in Figur 5: Spektrum d: 20131007-P6/2mm, Spektrum b: $TiO_2$-P25, Spektrum a: 20130912-P4: Probe 4, Spektrum c: 20130912-P3: Probe 3.

Alle auf ein Volumen bezogenen Angaben sind in der Maßeinheit Normliter. Der Plasmareaktor wurde bei 0,45 kW und etwa 14 kHz mit Hochspannungspulsen mit einer Halbwertsbreite, abgekürzt mit der dem Fachmann bekannten Angabe "*t(50)*", von 567 ns und einem Massestrom von 43 NL/min betrieben. Für die nachstehend genannten Plasma-Verfahren wurden im nicht-thermischen Gleichgewicht betriebene Gasentladungen verwendet.

Der in den Beispielen eingesetzte Free-Space Reaktor, abgekürzt "FSR", wies eine tangentiale Wandströmung auf. Der FSR war mit einer Anordnung zur Temperatur-Messung im Reaktor ausgestattet. Die Geometrie eines bevorzugten Reaktors ist nachfolgend wiedergegeben. Das Reaktor-Rohr wies einen Außendurchmesser von 36,1 mm auf, und einen Innendurchmesser von 33,1 mm. Die Heizzone war auf eine Länge von 700 mm bei einer Temperatur von 1300 °C vorgesehen.

Das Abschirmrohr für die Temperaturmess-Lanze wies einen Außendurchmesser von 6,7 mm, einen Innendurchmesser von 3,7 mm, und einen Messfühler für eine Messung hoher Temperaturen bis zu 2000 °C auf. Für eine Zirkulargasströmung im FSR war eine tangentiale Zuführung vorgesehen. Tabelle 1 Offenbart Messpunkte des Temperaturwertes im Rohreaktor.

Tabelle 1: Temperaturkurve in Abhängigkeit vom Messpunkt (x-Wert in mm) im Reaktor

| Messpunkt | X-Wert (mm) | Temperaturwert |
|-----------|-------------|----------------|
| M1 | -50 | RT |
| M2 | 0 | 595 |
| M3 | 50 | 926 |
| M4 | 100 | 1078 |
| M5 | 150 | 1149 |
| M6 | 200 | 1199 |
| M7 | 250 | 1227 |
| M8 | 300 | 1239 |
| M9 | 350 | 1238 |
| M10 | 400 | 1222 |
| M11 | 450 | 1186 |

(fortgesetzt)

| Messpunkt | X-Wert (mm) | Temperaturwert |
|---|---|---|
| M12 | 500 | 1120 |
| M13 | 550 | 929 |
| M14 | 600 | 553 |
| M15 | 650 | RT |

**Ausführungsbeispiel 1:** Gewinnung von amorphen Siliciumpartikeln in hochreiner Form in einem Free-Space Reaktor. Es wurde Monosilan in einer $H_2$-Matrix (60 Vol.-%) zersetzt. Der Wasserstoff wurde als Wärmeüberträger und als Verdünnungsgas verwendet. Die Verweilzeit in dem Reaktor-Rohr, das eine Länge von 50 cm aufwies, betrug 100 bis 400 Millisekunden.

Die Abkühlung und Abtrennung des amorphen Silicium erfolgt in einem Fluid. Vorliegend wurden die Zersetzungsprodukte durch flüssiges Paraffin geleitet. Die gebildeten amorphen Silicium Primärpartikel konnten im Paraffin stabilisiert werden und lagen je nach Herstellbedingungen und Konzentration in Form von Clustern aus aneinandergewachsenen Primärpartikeln vor.

Die Verfahrensbedingungen waren:

(a) Gasmischung 2 Normliter (NL/Min) Argon, 1 NL/Min Argon mit 5 Gew.-% $SiH_4$. Die Verweilzeit betrug 100 ms. Es wurde die Probe 3 erhalten.

(b) 2 NL/Min Argon und 2 NL/Min Argon mit 5 Gew.-% $SiH_4$. Die Verweilzeit betrug 400 ms. Es wurde die Probe 4 erhalten.

Es resultierten unterschiedliche Primärpartikelgrößen. Unter den Verfahrensbedingungen (a) wurde eine Primärpartikelgröße, dafür verwendet die dem Fachmann bekannte Abkürzung $d_{50}$, von 20 bis 25 nm erhalten. Unter den Bedingungen (b) resultierte eine Primärpartikelgröße von ($d_{50}$) von 35 bis 40 nm.

Die Absorption der amorphen Siliciumpartikel mit einer Partikelgröße von 20 bis 25 nm betrug im Bereich von 400 bis 1050 nm in einer Emulsion in Ethanol weniger als 1. Die Absorption sank bei Wellenlängen über etwa 500 nm auf unter 0,5 % ab und erreichte bei etwa 850 nm einen Wert von etwa 0,1 %. Die Partikelgrößen von (a) und (b) wiesen bei Wellenlängen unterhalb von 400 nm eine Absorption von größer gleich 1 auf. Die erhaltenen Silicium Primärpartikel waren im Wesentlichen amorph und je nach Primärpartikelgröße transparent.

Die Absorption der Partikel mit einer Partikelgröße von 35 bis 40 nm lag ab 400 nm unter 1 und verminderte sich auf 0,7 bei einer Wellenlänge von 1050 nm.

Die Absorption in Abhängigkeit von der Wellenlänge der erfindungsgemäß erhaltenen Partikel ist in Fig. 1b als die Linienform der Probe "*TiO$_2$ - P25*" gezeigt. Im Vergleich zu bekannten $TiO_2$ Partikeln zeigt das erfindungsgemäße nanopartikuläre Silicium mit Partikelgrößen von 20 bis 25 nm deutlich günstigere Transmissionseigenschaften, als $TiO_2$ Partikel im Wellenlängenbereich von 400 bis 740 nm und insbesondere bis 1100 nm.

Es wurde außerdem festgestellt, dass durch Dotieren des nanopartikulären Siliciums mit Selen die erfindungsgemäßen Partikel eine Wirkung als Radikalfänger aufweisen.

**Ausführungsbeispiele 2a und 2b: Plasma**

Die Verfahrensbedingungen waren wie im Ausführungsbeispiel 1, jedoch mit folgenden Unterschieden.

(2a) Es wurde Monosilan zusammen mit etwa 10 ppm Selen eingesetzt. Das nicht-thermische Plasma wies eine Matrix aus Argon und 1% Xenon auf.

Xenon begünstigte die Homogenität der Entladung im Reaktorraum.

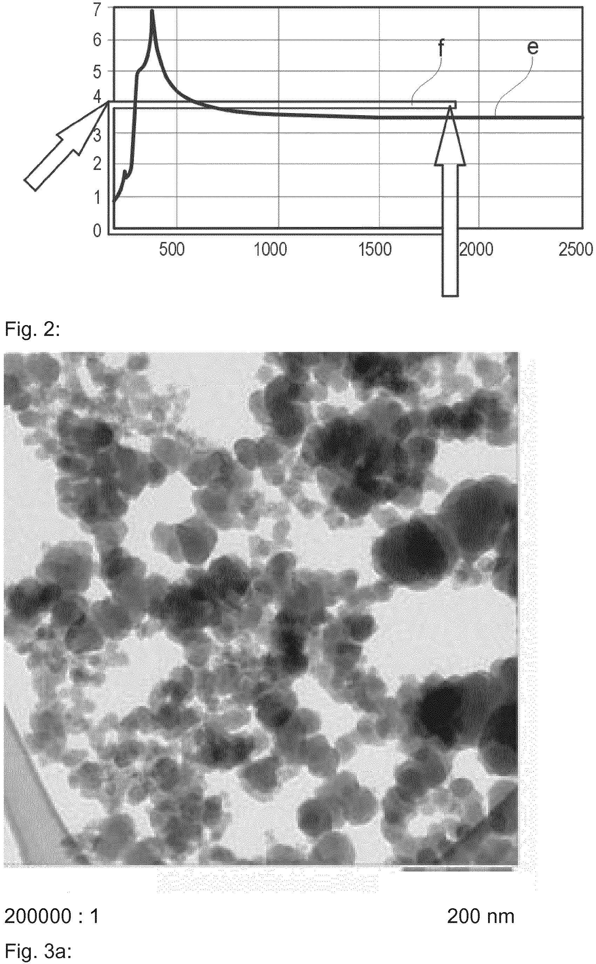

Die mittlere Primärpartikelgröße der erfindungsgemäß erhaltenen Silicium aufweisenden Partikel lag bei 5 bis 10 nm. Fig. 3a zeigt eine TEM Aufnahme dieser Partikel.

(2b) wie (2a), jedoch mit der Zugabe von 1 Vol.-% Sauerstoff und verlängerten Verweilzeiten.

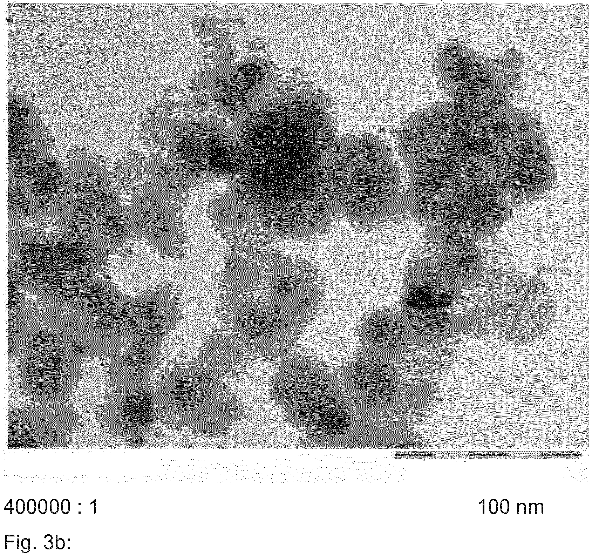

Die mittlere Primärpartikelgröße der erfindungsgemäß erhaltenen Silicium aufweisenden Partikel, die eine $SiO_2$ Shell aufwiesen, lag bei etwa 40 nm. Fig. 3b zeigt eine TEM Aufnahme dieser Partikel.

**Patentansprüche**

1. Verwendung von Silicium enthaltenden Partikeln,
**dadurch gekennzeichnet, dass** die Partikel zum Schutz von Zellen vor elektromagnetischer Strahlung im Wellenlängenbereich von 10 bis 1500 nm verwendet werden.

2. Verwendung nach Anspruch 1,
**dadurch gekennzeichnet, dass**
die Partikel

   a) zur Absorption von elektronmagnetischer Strahlung im Wellenlängenbereich von größer gleich 10 nm bis 1100 nm verwendet werden, insbesondere im Wellenlängenbereich von 10 nm bis 450 nm,
   b) als UV-Schutz vor elektronmagnetischer Strahlung verwendet werden.

3. Verwendung nach Anspruch 1 oder 2,
**dadurch gekennzeichnet, dass**
die Silicium enthaltenden Partikel einen Gehalt von größer gleich 50 bis 100 Gew.-% Silicium und eine Primärpartikelgröße von 1 bis 500 nm aufweisen.

4. Verwendung nach einem der Ansprüche 1 bis 3,
**dadurch gekennzeichnet, dass**
die Silicium enthaltenden Partikel im Wesentlichen amorphe Partikel sind.

5. Verwendung nach einem der Ansprüche 1 bis 4,
**dadurch gekennzeichnet, dass**
die Silicium enthaltenden Partikel im Wesentlichen amorphe Partikel aus Silicium sind.

6. Verwendung nach einem der Ansprüche 1 bis 5,
**dadurch gekennzeichnet, dass**
die Silicium Partikel eine Kristallinität von kleiner gleich 2 % aufweisen.

7. Verwendung nach einem der Ansprüche 1 bis 5,
**dadurch gekennzeichnet, dass**
die Silicium enthaltenden Partikel

   a) Primärpartikel einer Größe von 1 nm bis 200 nm aufweisen, insbesondere weisen sie Primärpartikel von 5 bis 100 nm auf, und/oder
   b) eine mittlere Primärpartikelgröße von im Mittel $d_{50}$ = 15 bis 40 nm.

8. Verwendung nach einem der Ansprüche 1 bis 7,
**dadurch gekennzeichnet, dass**
die Partikel zu 60 bis 100 Gew.-% Silicium umfassen und auf 100 Gew.-% einen Gehalt an Kohlenstoff und optional Sauerstoff aufweisen und optional transparent sind.

9. Verwendung nach einem der Ansprüche 1 bis 8,
**dadurch gekennzeichnet, dass**
die Partikel die nachfolgende Absorptionscharakteristika von elektromagnetischer Strahlung aufweisen

   a) mit einer Primärpartikelgröße von 10 bis 50 nm, insbesondere mit $d_{50}$ um 35 nm, ist die Absorption im Wellenlängenbereich von 250 bis 400 im Vergleich zur Absorption im Wellenlängenbereich von 400 bis 750 nm mit einer Abweichung von +/- 40 % gleich, und/oder
   b) mit einer Primärpartikelgröße von 10 bis 50 nm, insbesondere mit $d_{50}$ um 25 nm, ist das Verhältnis der Absorption im Wellenlängenbereich von (i) 250 bis 400 im Vergleich zur Absorption bei einer Wellenlänge von (ii) 550 nm etwa (i) zu (ii) von 2 : 1 bis 8 : 1 mit einer Abweichung von +/- 40 %, und/oder
   c) mit einer Primärpartikelgröße von 10 bis 50 nm, insbesondere mit $d_{50}$ um 25 nm, ist das Verhältnis der Absorption im Wellenlängenbereich von (i) 250 bis 400 im Vergleich zur Absorption bei einer Wellenlänge von (ii) 500 nm bei etwa (i) zu (ii) von 1,5 : 1 bis 8 : 1 mit einer Abweichung von +/- 30 %, und/oder
   d) mit einer Primärpartikelgröße von 10 bis 50 nm, insbesondere mit $d_{50}$ um 25 nm, ist das Verhältnis der Absorption im Wellenlängenbereich von (i) 250 bis 350 nm im Vergleich zur Absorption bei einer Wellenlänge von (ii) 450 nm etwa (i) zu (ii) von 2 : 1 bis 4 : 1 mit einer Abweichung von +/- 30 %, und/oder

e) mit einer Primärpartikelgröße von 10 bis 50 nm, insbesondere mit $d_{50}$ um 25 nm, ist das Verhältnis der Absorption bei einer Wellenlänge von (i) 250 nm im Vergleich zur Absorption bei einer Wellenlänge von (ii) 450 nm bei etwa (i) zu (ii) von 2 : 1 bis 4 : 1, mit einer Abweichung von +/- 30 %, und/oder

f) mit einer Primärpartikelgröße von 10 bis 50 nm, insbesondere mit $d_{50}$ um 25 nm, ist das Verhältnis der Absorption bei einer Wellenlänge von (i) 350 nm im Vergleich zur Absorption bei einer Wellenlänge von (ii) 450 nm bei etwa (i) zu (ii) von 2 : 1 bis 3 : 1, mit einer Abweichung von +/- 30 %.

10. Verwendung nach einem der Ansprüche 1 bis 9,
**dadurch gekennzeichnet, dass**
die Silicium enthaltenden Partikel eine Brechzahl (n) von größer gleich 3,0 bei einer Wellenlänge von 500 bis 2500 nm und/oder eine Brechzahl (n) von größer gleich 4,0 bei einer Wellenlänge von 200 bis 500 nm aufweisen.

11. Verwendung, insbesondere nach einem der Ansprüche 1 bis 10,
**dadurch gekennzeichnet, dass**
die Silicium enthaltenden Partikel zur Erhöhung der Brechzahl einer kosmetischen, dentalen, medizinischen oder pharmazeutischen Formulierung verwendet werden.

12. Verwendung von Silicium enthaltenden Partikeln
umfassend Primärpartikel von 5 bis 100 nm und optional Cluster dieser Primärpartikel, mit einem Gehalt an Silicium von größer gleich 90 Gew.-% bis 100 Gew.-% als biologisch verträglicher UV-Schutz und/oder als biologisch abbaubarer UV-Schutz.

13. Verfahren zur Herstellung von Silicium enthaltenden Partikeln,
insbesondere zur Verwendung nach einem der Ansprüche 1 bis 12,
indem

a) mindestens eine gasförmige oder bei erhöhter Temperatur gasförmige Siliciumverbindung,
b) optional in Gegenwart mindestens eines unter den Reaktionsbedingungen reaktiven Gases oder einer Mischung von reaktiven Gasen,
c) in Gegenwart eines Verdünnungsgases in einer im Wesentlichen sauerstofffreien Atmosphäre unter (i) thermischen Bedingungen und/oder (ii) im Plasma zersetzt wird, und
d) mit einem Fluid als Silicium enthaltende Partikel abgeschieden wird.

14. Verfahren nach Anspruch 13,
**dadurch gekennzeichnet, dass**
die Silicium enthaltenden Partikel mit einem Fluid in Form einer Lösung, Emulsion, Suspension, Gel, Schaums, Aerosols, Rauch oder kolloidal in einer Lösung abgeschieden werden.

15. Verfahren nach Anspruch 13 oder 14,
**dadurch gekennzeichnet, dass**
die gasförmige Siliciumverbindung Wasserstoff enthaltende Silane umfasst, wie Monosilan, Disilan, Trisilan und Gemische enthaltend mindestens eines der Silane.

16. Formulierung enthaltend amorphe Silicium enthaltende Partikel mit einen Gehalt von größer gleich 50 bis 100 Gew.-% Silicium und einem Gehalt an Kohlenstoff und optional Sauerstoff und einer mittleren Primärpartikelgröße von 5 bis 500 nm, wobei die Partikel vorzugsweise als Cluster von Primärpartikeln vorliegen, oder Silicium enthaltende Partikel erhalten nach einem Verfahren nach einem der Ansprüche 13 bis 15,
**dadurch gekennzeichnet, dass**
sie eine kosmetische, medizinische oder pharmazeutische Formulierung für den UV-Schutz der Haut oder der Haare ist, insbesondere eine Sonnenschutzcreme, Sonnenschutzgel, Sonnenschutzöl, Sonnenschutzspray.

17. Formulierung nach Anspruch 16,
**dadurch gekennzeichnet, dass**
sie mindestens ein Hilfsmittel umfasst.

Fig. 1a

Fig. 1b

Fig. 2:

200000 : 1                                             200 nm

Fig. 3a:

400000 : 1                                                    100 nm

Fig. 3b:

Europäisches
Patentamt

European
Patent Office

Office européen
des brevets

**EUROPÄISCHER RECHERCHENBERICHT**

Nummer der Anmeldung

EP 14 19 5302

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | ALEXANDER V. PAVLIKOV ET AL: "Optical properties of materials based on oxidized porous silicon and their applications for UV protection", MICROELECTRONIC ENGINEERING, Bd. 90, 1. Februar 2012 (2012-02-01), Seiten 96-98, XP55187893, ISSN: 0167-9317, DOI: 10.1016/j.mee.2011.06.005 * das ganze Dokument * | 1-15 | INV. A61K8/25 A61Q17/00 A61Q17/04 C01B33/027 C01B33/029 C01B33/03 |
| X | WO 99/32574 A1 (BAYER AG [DE]; BERNETH HORST [DE]; DUFF DANIEL GORDON [DE]; HOHEISEL W) 1. Juli 1999 (1999-07-01) | 1-5,8, 11-15 | |
| Y | * Seite 5, Zeile 5 * * Seite 8, Zeile 18 - Seite 9, Zeile 16 * * Seite 9, Zeile 24 - Seite 10, Zeile 2 * * Seite 7, Zeilen 1-13 * | 1-15 | |

RECHERCHIERTE SACHGEBIETE (IPC)

A61K
A61Q
C01B

Der vorliegende Recherchenbericht wurde für alle Patentansprüche erstellt

| Recherchenort | Abschlußdatum der Recherche | Prüfer |
|---|---|---|
| München | 8. Mai 2015 | Uhl, Martin |

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 14 19 5302

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-05-2015

| Im Recherchenbericht angeführtes Patentdokument | Datum der Veröffentlichung | Mitglied(er) der Patentfamilie | Datum der Veröffentlichung |
|---|---|---|---|
| WO 9932574 A1 | 01-07-1999 | AT 268805 T | 15-06-2004 |
| | | AU 741615 B2 | 06-12-2001 |
| | | AU 2268499 A | 12-07-1999 |
| | | BR 9813759 A | 03-10-2000 |
| | | CA 2315080 A1 | 01-07-1999 |
| | | DE 19756740 A1 | 24-06-1999 |
| | | EP 1042427 A1 | 11-10-2000 |
| | | ES 2223146 T3 | 16-02-2005 |
| | | JP 2001527221 A | 25-12-2001 |
| | | PL 341100 A1 | 26-03-2001 |
| | | TW 557383 B | 11-10-2003 |
| | | US 6466355 B1 | 15-10-2002 |
| | | WO 9932574 A1 | 01-07-1999 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **A.T. BELL.** Fundamentals of Plasma Chemistry. Wiley, 1974

- Plasmatechnik: Grundlagen und Anwendungen - Eine Einführung; Autorenkollektiv. Carl Hanser Verlag, 1984